# EUROPÄISCHE PATENTSCHRIFT

(11) **EP 0 326 023 B1**
(45) Veröffentlichungstag und Bekanntmachung des Hinweises auf die Patenterteilung: **26.10.1994**
(21) Anmeldenummer: 89100901.1
(22) Anmeldetag: 19.01.1989
(51) Int. Cl.: A61K 31/535

(54) **Benzamid gegen kognitive Erkrankungen**
Benzamide against cognitive disorders
Benzamide contre des maladies cognitives

(30) Priorität: 28.01.1988 CH 298/88
(43) Veröffentlichungstag der Anmeldung: 02.08.1989
(73) Patentinhaber: F. HOFFMANN-LA ROCHE AG, 4002 Basel (CH)
(72) Erfinder: Amrein, Roman, Dr., CH-4126 Bettingen (CH); Anand, Ravi, Dr., CH-4058 Basel (CH)
(74) Vertreter: Lederer, Franz, Dr.

(56) Entgegenhaltungen:
- NEUROPSYCHOBIOLOGY, Band 15, Nr. 3-4, 1986, Seiten 133-142, Karger AG, Basel,CH; G.M. WHITFORD: "Alzheimer's disease and serotonin: A review"
- ACTA THERAPEUTICA, Band 11, 1985, Seiten 249-252; J.U. POSTMA et al.:"Moclobemide in the treatment of depression in demented geriatric patients"
- BR. J. CLIN. PHARMACOL., Band 27, Nr. 5, 1984, Seiten 647P-648P, Proceedings ofthe BPS, 19. - 21. Dezember 1988; K.A. WESNES et al.: "Acute cognitive effectsof moclobemide and trazodone, alone and in combination with alcohol, in the
- elderly"
- SEM. HOP. PARIS, Band 61, Nr. 31, 1985, Seiten 2311-2315; D. MALAUZAT et al.:"Intérét des benzamides substitués en gériatrie"
- SEM. HOP. PARIS, Band 57, Nr. 43-44, 1981, Seiten 1833-1836; G. MILETTO et al.:"Le tiapride en neurologie et en psychiatrie chez la personne âgée"
- PHARMACOPSYCHIAT., Band 17, 1984, Seiten 122-125, Georg Thieme Verlag,Stuttgart, DE; M. CASSOCHIA et al.: "A placebo-controlled study of theantidepressant activity of moclobemide, a new MAO-A inhibitor"
- J. PHARM. AND PHARMACOL., Band 36, Suppl. 64W, 1984, Seite 64W; A. KORN et al.:"Moclobemide, a new MAO-A inhibitor, does not provoke a "cheese reaction" afteringestion of tyramine containing meals"
- J. NEURAL TRANSM., Band 28, 1989, Seiten 91-102, Springer-Verlag; K.A. WESNESet al.: "The effects of moclobemide on cognition"
- ADVANCES IN NEUROLOGY, Band 51, 1990, Seiten 261-268, Raven Press, Ltd, New
- York, US; R. ANAND et al.: "Cognition-enhancing effects of moclobemide, areversible MAO inhibitor, in humans"
- Arzneimittel-Kompendium der Schweiz 1993 (Seite 124, 1442,1167,414)

## Beschreibung

Im Rahmen der vorliegenden Erfindung wurde festgestellt, dass das p-Chlor-N-(2-morpholinoäthyl)benzamid (nachstehend Moclobemid genannt) der Formel 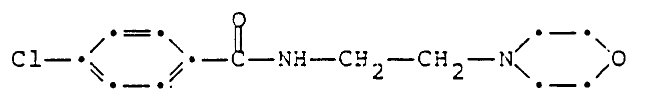
kognitiven Erkrankungen entgegenzuwirken vermag. Bei Moclobemid handelt es sich um einen reversiblen MAO-A-Hemmer, der zur Behandlung von depressiven Zuständen verwendet wird. Es kann in diesem Zusammenhang auf die folgenden Patentschriften und Literaturstellen verwiesen werden:
Deutsche Offenlegungsschrift 2 706 179
U.S. Patentschrift 4 210 754
Pharmacopsychiat., 17, (1984), 122-125
J. Pharm. and Pharmacol., Vol. 36, Suppl., November 1984, 64 W
Acta Therapeutica, 11, (1985), 249-251
In Sem. Hop. Paris, Band 61 (31) S. 2311-5, 1985 wird erwähnt, dass Neuroleptika wie Metoclopramid, Sulpirid und Tiaprid zur Behandlung von u.a. das Gedächtnis beeinflussenden mentalen Erkrankungen eingesetzt werden können. In Sem. Hop. Paris Band 57 (43-44) S. 1833-6, 1981 wird erwähnt, dass Tiaprid bei der Behandlung der Alzheimer-Erkrankung unwirksam war.

Aus Acta Therapeutica, l.c., geht hervor, dass Moclobemid zur Behandlung von Depressionen bei geriatrischen Patienten mit altersbedingter Demenz verwendet wurde, wobei bei allen Patienten eine vernünftige oder gar substantielle Verbesserung der Depressionssymptome erreicht wurde. Dass die Depressionssymptome nur in einigen wenigen Fällen vollständig zum Abklingen gebracht werden konnten, erklären die Autoren damit, dass die Demenz wahrscheinlich bestehen blieb. Ueberraschend wurde nun aber gefunden, dass durch Verabreichung von Moclobemid kognitive Erkrankungen durchaus erfolgreich behandelt bzw. verhütet werden können. Die vorliegende Erfindung basiert auf dem eben genannten Befund und betrifft demnach die neue Verwendung von Moclobemid bei der Behandlung bzw. Verhütung von kognitiven Erkrankungen, insbesondere von solchen, die altersbedingt sind. Beispiele solcher Erkrankungen sind altersbedingte Gedächtnisschwäche, primäre und sekundäre degenerative Demenz, z.B. Demenz vom Alzheimer-Typ oder multi-infarktbedingte Demenz, und cerebrovaskuläre Erkrankungen und Folgen von Hirnschlägen.

Zur Behandlung bzw. Verhütung der oben genannten Krankheiten wird Moclobemid systemisch, vorzugsweise enteral, besonders bevorzugt oral, verabreicht.

Die Dosis variiert gemäss den Bedürfnissen des einzelnen Patienten, wie sie vom behandelnden Arzt bestimmt worden sind. Im allgemeinen dürfte jedoch eine tägliche Dosis von 0,1 mg bis 20 mg, vorzugsweise von 1 mg bis 10 mg, pro kg Körpergewicht des Patienten zum Einsatz gelangen. Die Dosis kann als Einzeldosis oder auf mehrere Teildosen verteilt gemäss einem Dosierungsplan, wie er vom Arzt gemäss den Bedürfnissen des Patienten bestimmt wird, verabreicht werden.

Als Verabreichungsform kommen die für die systemische Verabreichung üblichen festen oder flüssigen Darreichungsformen in Betracht, z.B. Suppositorien oder, als feste orale Darreichungsformen, Kapseln, Tabletten, Lacktabletten, Dragées, Pillen, Puder, Granulate und dergleichen, als flüssige orale Darreichungsformen Lösungen, Sirupe, Suspensionen, Elixiere und dergleichen und als parenterale Darreichungsformen Infusions- oder Injektionslösungen, die intravenös oder intramuskulär injiziert werden können.

Es liegt im Bereich der vorliegenden Erfindung, Moclobemid in jeder für die Verabreichung geeigneten Menge in die enterale oder parenterale Darreichungsform einzuarbeiten. Es ist jedoch bevorzugt, Präparate herzustellen, die den erfindungsgemässen Wirkstoff in einer Menge von 5-500 mg, vorzugsweise von 50-200 mg, enthalten. Besonders bevorzugt ist die Herstellung von Kapseln und Lacktabletten.

Die Herstellung der oben genannten Gebrauchsformen kann in üblicher Weise, z.B. anhand der nachstehenden Beispiele erfolgen.

### Beispiel 1

Lacktabletten, enthaltend die folgenden Bestandteile, können hergestellt werden:

| Bestandteile | mg/Lacktablette |
|---|---|
| 1. Moclobemid | 50,0 |
| 2. Milchzucker pulv. | 74,0 |
| 3. Maisstärke | 60,0 |
| 4. Polyvinylpyrrolidon | 10,0 |
| 5. Natriumcarboxymethyl-Stärke | 5,0 |
| 6. Magnesiumstearat | 1,0 |
| Total | 20̅0̅,̅0̅ |

### Verfahren:

### a) Herstellung der unlackierten Tabletten

Das Moclobemid wird mit dem Milchzucker pulv., der Maisstärke und dem Polyvinylpyrrolidon gemischt und gesiebt. Die Pulvermischung wird mit entmineralisiertem Wasser befeuchtet und geknetet, und die resultierende Masse granuliert, getrocknet und gesiebt. Das Granulat wird mit der gesiebten Natriumcarboxymethyl-Stärke und dem gesiebtem Magnesiumstearat vermischt und zu Tabletten verpresst.

### b) Lackierung der Tabletten

2,5 mg Hydroxypropylmethylcellulose werden in 25 mg entmineralisiertem Wasser gelöst. In diese Lösung wird eine wassrige (10 mg) Suspension von 1,88 mg Talk, 0,5 mg Titandioxyd, 0,1 mg Eisenoxyd gelb und 0,02 mg Eisenoxyd rot eingerührt. Die Lacksuspension wird auf die Tabletten gesprüht, und die Lacktabletten über Nacht bei 45°C getrocknet. Das Gesamtgewicht einer Lacktablette beträgt 205,0 mg.

### Beispiel 2

Lacktabletten, enthaltend die folgenden Bestandteile, können hergestellt werden:

| Bestandteile | mg/Lacktablette |
|---|---|
| 1. Moclobemid | 100,0 |
| 2. Milchzucker pulv. | 148,0 |
| 3. Maisstärke | 120,0 |
| 4. Polyvinylpyrrolidon | 20,0 |
| 5. Natriumcarboxymethyl-Stärke | 10,0 |
| 6. Magnesiumstearat | 2,0 |
| Total | 4̅0̅0̅,̅0̅ |

### Verfahren:

### a) Herstellung der unlackierten Tabletten

Das Moclobemid wird mit dem Milchzucker pulv., der Maisstärke und dem Polyvinylpyrrolidon gemischt und gesiebt. Die Pulvermischung wird mit entmineralisiertem Wasser befeuchtet und geknetet, und die resultierende Masse granuliert, getrocknet und gesiebt. Das Granulat wird mit der gesiebten Natriumcarboxymethyl-Stärke und dem gesiebtem Magnesiumstearat vermischt und zu Tabletten verpresst.

### b) Lackierung der Tabletten

5 mg Hydroxypropylmethylcellulose werden in 50 mg entmineralisiertem Wasser gelöst. In diese Lösung wird eine wässrige (20 mg) Suspension von 3,76 mg Talk, 1,0 mg Titandioxyd, 0,2 mg Eisenoxyd gelb und 0,04 mg Eisenoxyd rot eingerührt. Die Lacksuspension wird auf die Tabletten gesprüht, und die Lacktabletten über Nacht bei 45°C getrocknet. Das Gesamtgewicht einer Lacktablette beträgt 410,0 mg.

### Beispiel 3

Lacktabletten, enthaltend die folgenden Bestandteile, können hergestellt werden:

| Bestandteile | mg/Lacktablette |
|---|---|
| 1. Moclobemid | 150,0 |
| 2. Milchzucker pulv. | 148,0 |
| 3. Maisstärke | 60,0 |
| 4. Polyvinylpyrrolidon | 25,0 |
| 5. Natriumcarboxymethyl-Stärke | 15,0 |
| 6. Magnesiumstearat | 2,0 |
| Total | 4̅0̅0̅,̅0̅ |

### Verfahren:

### a) Herstellung der unlackierten Tabletten

Das Moclobemid wird mit dem Milchzucker pulv., der Maisstärke und dem Polyvinylpyrrolidon gemischt und gesiebt. Die Pulvermischung wird mit entmineralisiertem Wasser befeuchtet und geknetet, und die resultierende Masse granuliert, getrocknet und gesiebt. Das Granulat wird mit der gesiebten Natriumcarboxymethyl-Stärke und dem gesiebtem Magnesiumstearat vermischt und zu Tabletten verpresst.

### b) Lackierung der Tabletten

4,5 mg Hydroxypropylmethylcellulose werden zusammen mit 0,6 mg Polyäthylenglykol in 55 mg entmineralisiertem Wasser gelöst. In diese Lösung wird eine wässrige (41 mg) Suspension von 2,4 mg Talk, 2,9 mg Titandioxyd und 0,1 mg Eisenoxyd gelb eingerührt. Nach Zugabe von 1,5 mg Aethylcellulose-Dispersion erhält man die Lacksuspension, welche auf die Tabletten gesprüht wird. Die Lacktabletten werden über Nacht bei 45°C getrocknet. Das Gesamtgewicht einer Lacktablette beträgt 412,0 mg.

### Beispiel 4

Sachets, enthaltend die folgenden Bestandteile, können hergestellt werden:

| Bestandteile | mg/Sachet |
|---|---|
| 1. Moclobemid | 400,0 |
| 2. Milchzucker pulv. | 1870,0 |
| 3. Maisstärke weiss | 680,0 |
| 4. Polyvinylpyrrolidon | 50,0 |
| Total | 30̅0̅0̅,̅0̅ |

### Verfahren:

Die oben genannten Bestandteile werden gemischt. Die Mischung wird mit ca. 800 mg Wasser befeuchtet, und die resultierende Masse granuliert, getrocknet und gesiebt.

### Beispiel 5

Hartgelatinekapseln, enthaltend die folgenden Bestandteile, können hergestellt werden:

| Bestandteile | mg/Kapsel |
|---|---|
| 1. Moclobemid | 200,0 |
| 2. Milchzucker pulv. | 162,0 |
| 3. Maisstärke weiss | 100,0 |
| 4. Polyvinylpyrrolidon | 8,0 |
| 5. Talk | 9,0 |
| 6. Magnesiumstearat | 1,0 |
| Total | 4̅8̅0̅,̅0̅ |

### Verfahren:

Das Moclobemid wird mit dem Milchzucker pulv., der Maisstärke und dem Polyvinylpyrrolidon gemischt. Die Mischung wird mit entmineralisiertem Wasser befeuchtet und geknetet, und die resultierende Masse granuliert, getrocknet und gesiebt. Das erhaltene Granulat wird mit dem Talk und dem Magnesiumstearat vermischt, und das erhaltene Pulver in Steckkapseln abgefüllt.

Die therapeutische und prophylaktische Wirkung von Moclobemid bei kognitiven Erkrankungen kann den nachfolgenden Versuchen entnommen werden:
A) Die die cerebrale Insuffizienz inhibierende Wirkung von Moclobemid wurde nach dem folgenden Prozedere an der weiblichen Albinoratte (Füllinsdorf/Schweiz) bestimmt. Die Testapparatur ist eine Skinnerbox mit einem elektrifizierbaren Gitterboden und einer grauen Viereckplattform in einer Ecke. Unerfahrene weibliche Ratten mit einem Gewicht von 100-120 g werden einzeln auf die Plattform gebracht. Sobald sie auf den Gitterboden hinabsteigen, erhalten sie einen elektrischen Fusschock (0,8 mA). Die normale Reaktion unerfahrener Ratten ist, daraufhin auf die Plattform zurückzuspringen. Da jedoch die Ratten immer nochmals herabzuklettern versuchen, muss die Fusschockprozedur für jedes Tier mehrmals wiederholt werden. Nach diesen mehrmaligen Wiederholungen pro Tier haben die Ratten ein sogenanntes "passives Vermeidungsverhalten" erlernt, d.h. sie versuchen nicht mehr, auf den Gitterboden hinabzusteigen, weil sie wissen, dass sie mit einem Elektroschock bestraft werden.
   Unmittelbar anschliessend werden die Ratten in Gruppen von je mindestens 15 Tieren eingeteilt. Die erste Gruppe erhält eine Injektion (s.c.) von 1 mg/kg Scopolamin-hydrobromid sowie eine orale Dosis des Trägermaterials, während die übrigen Gruppen eine Injektion (s.c.) von 1 mg/kg Scopolamin-hydrobromid und verschiedene orale Dosen Moclobemid erhalten.
   Zwei Stunden später wird jede Ratte einmal auf die Plattform in der Skinnerbox gesetzt. Das Kriterium für die Beurteilung dieses Tests zur Ermittlung einer Präparatwirkung auf das Kurzzeit-Gedächtnis ist, ob das Tier während ca. 30 Sekunden auf der Plattform bleibt oder nicht (das Ergebnis kann also für jedes Tier nur "ja" oder "nein" lauten). Die statistische Signifikanz der Unterschiede zwischen den bei der ersten und den übrigen Gruppen erhaltenen Resultaten wird mittels des Chi-Quadrat-Tests ermittelt. Eine Dosis Moclobemid wird dann als aktiv bezeichnet, wenn die Anzahl der positiven Ergebnisse ("ja") von der bei den mit Scopolamin-hydrobromid (1 mg/kg s.c.) und nur Placebo (p.o.) behandelten Kontrolltieren signifikant verschieden ist.
   In diesem Test bewirken orale Dosen von 0,1, 0,3 und 1 mg/kg Moclobemid einen signifikanten Anstieg der positiven Ergebnisse im Vergleich zu den Ergebnissen bei den mit Scopolamin-hydrobromid und nur Placebo behandelten Kontrolltieren.
B) Zur Untersuchung der Wirkung auf das Langzeit-Gedächtnis wird die oben beschriebene Versuchsanordnung mit den folgenden zwei Abweichungen durchgeführt. Erstens werden den Versuchstieren anstelle von 1 mg/kg Scopolamin-hydrobromid 3 mg/kg Cycloheximid subcutan verabreicht, und zweitens erfolgt die Beurteilung zur Ermittlung einer Präparatwirkung erst 48 Stunden später.

In diesem Test zeigt Moclobemid in einer oralen Dosis von 1 mg/kg eine signifikante Aktivität.

Die obigen, im Tierversuch ermittelten Ergebnisse konnten im nachfolgend beschriebenen humanexperimentellen Versuch bestätigt werden, welcher in Uebereinstimmung mit der Deklaration von Helsinki, in der Fassung von Venedig, durchgeführt wurde.

29 junge, gesunde, normale männliche Probanden, die vorgängig des Versuchs ihr schriftliches Einverständnis dazu gegeben hatten, beendeten diese Studie. Damit kein Proband ein gesundheitliches Risiko eingehen musste, wurden diese nach äusserst strengen Kriterien ausgewählt und während des Experiments einer gründlichen medizinischen Untersuchung, inklusive Bestimmung der hämatologischen Werte, einer biochemischen Analyse von Blut und Urin sowie einem EEG, unterworfen. Da es sich bei der Testsubstanz um einen MAO-Hemmer handelt, wurde den Probanden an allen Versuchstagen sowie den Tagen davor und danach eine strenge Diät auferlegt. Jeder Proband musste in wöchentlichem Abstand den Versuch mit jeweils einer der verschiedenen Testsubstanzen absolvieren. Nach Beendigung des Experiments wurden alle Probanden erneut einer weiteren gründlichen ärztlichen Untersuchung unterworfen.

Um die Beeinträchtigung der kognitiven Leistungsfähigkeit nach Verabreichung von Scopolamin sowie deren mögliche Verbesserung nach nachträglicher Verabreichung der Testverbindungen zu erfassen, wurde eine Batterie psychometrischer Tests zusammengestellt, die im wesentlichen aus folgenden Untertests bestand (alle hierbei verwendeten Verfahren haben sich als sensitiv bezüglich Scopolamin erwiesen):
1. "Buschke-Fuld Selective Reminding Task" (Buschke und Fuld, Neurology, 1974, 24, 1019-1025). Dieses Verfahren hat sich als sensitiv zur Erfassung der kognitiven Defizite bei Alzheimerpatienten erwiesen und spiegelt deren Defizite wieder (Ober et al., Brain and Cognition 1985, 4, 90-103).
2. "Zahlen-verbindungs-Test (Trail Making Task)" (Reitan, Perceptual und Motor Skills 1958, 8, 71-76; Oswald und Fleischmann, Nürnberger Altersinventar 1986). Hierbei handelt es sich um ein Verfahren, um speziell visuomotorische Koordinationsfähigkeit sowie Aufmerksamkeit zu erfassen. Gerade bei Patienten mit organisch bedingten Hirnschäden hat sich dieser Test zur Erfassung der Leistungsbeeinträchtigung als besonders sensitiv erwiesen.
3. "Wahl-Reaktionszeiten (Choice Reaction Time)" wurden seit vielen Jahren immer wieder zur Untersuchung der Beeinflussung psychomotorischer Leistungen durch Medikamente verwendet (z.B. Hindmarch, Brit. J. Clin. Pharmac. 1979, 8, 43S-46S). Sie stellen ein gutes Mass der allgemeinen kognitiven Leistungsfähigkeit dar.
4. "Sternberg Memory Scanning" (Sternberg, Q. J. of Exp. Psychol., 1975, 27, 1-32) ist ebenfalls ein Medikamenten-sensitives Verfahren (Subhan und Hindmarch, Neuropsychobiology 1984, 12, 244-248) zur Erfassung der Leistung des Kurzzeit-Gedächtnisses.
5. "Rapid Visual Information Processing Task" (z.B. Wesnes und Warburton, Psychopharmacology, 1984, 82, 147-150), ist ein Medikamenten-sensitives Verfahren und dient zur Messung der längerfristigen Konzentrationsfähigkeit.

Die Probanden konnten vor Beginn der Studie Parallelformen der Testbatterie zur Erfassung kognitiver Leistungen trainieren und erfüllten die an sie gestellten Anforderungen mindestens bei drei Gelegenheiten. Die Probanden wurden aufgefordert, 12 Stunden vor dem jeweiligen Versuchstag bis zum Abschluss der Datenerhebung nicht zu rauchen und keine koffeinhaltigen Getränke zu sich zu nehmen.

Der Versuch begann damit, dass die Probanden zunächst die Testbatterie vor Verabreichung irgendeiner Substanz absolvieren mussten, um die Basiswerte zu erhalten, die dann mit den 60 Minuten nach Verabreichung des Scopolamins erhaltenen Werten verglichen wurden, um den durch Scopolamin induzierten Grad der Beeinträchtigung festzustellen. Eine Bedingung war, dass die durch Scopolamin induzierte Beeinträchtigung des Gedächtnisses im Buschke-Fuld-Test 60 Minuten nach Verabreichung von Scopolamin bezüglich des Abrufs aus dem Langzeit-Gedächtnis mindestens 30% betrug.

Nach Bestimmung der Basiswerte wurde jedem Probanden 0,7 mg Scopolamin-hydrochlorid subcutan injiziert. 60 Minuten später hatte der Proband die Testbatterie wiederum zu absolvieren, um jetzt die durch Scopolamin induzierte Beeinträchtigung festzustellen. 95 Minuten nach der Scopolamin-Injektion wurde den Probanden eine Testsubstanz in 400 ml Orangensaft oral verabreicht. Bei den Testsubstanzen handelt es sich um 1500 mg Aniracetam, 750 mg, 1500 mg bzw. 3000 mg 3-Hydroxyaniracetam, 400 mg Moclobemid bzw. Placebo. 120 Minuten nach der Scopolamin-Injektion mussten die Probanden wiederum die Testbatterie absolvieren.

Für jeden Untertest wurden die 60 Minuten nach der Scopolamin-Injektion erhaltenen Werte als Basiswerte genommen. Für jeden Probanden wurde dieser Wert vom entsprechenden Wert subtrahiert, der 120 Minuten nach der Scopolamin-Injektion, d.h. 35 Minuten nach Verabreichung einer Testsubstanz, erhalten wurde, wobei die Differenz ein Mass für die durch die orale Verabreichung der Testsubstanz erwirkten Antagonisierung des durch Scopolamin induzierten Effektes darstellt.

Eine globale Analyse wurde zur Feststellung der Gesamtwirkung der verschiedenen Testsubstanzen durchgeführt. Hierbei wurde eine Rangreihe bezüglich der Leistungsverbesserung nach Verabreichung der Testsubstanzen gegenüber den nach der Scopolamin-Injektion erhaltenen Testwerte über die sechs experimentellen Bedingungen (Testsubstanzen) erstellt. Die dabei erhaltenen Resultate präsentieren sich wie folgt, wobei die höchste Rangsumme die beste Leistungsverbesserung darstellt:

| | | |
|---|---|---|
| Placebo | | 95 |
| Moclobemid | | 122,5 |
| Aniracetam | | 108,5 |
| 3-Hydroxyaniracetam | 750 mg | 94 |
| | 1500 mg | 95,5 |
| | 3000 mg | 93,5 |

## Patentansprüche

1. Verwendung von p-Chlor-N-(2-morpholinoäthyl)benzamid zur Herstellung eines Mittels zur Behandlung bzw. Verhütung von kognitiven Erkrankungen.

2. Verwendung gemäss Anspruch 1, dadurch gekennzeichnet, dass es sich um eine altersbedingte kognitive Erkrankung handelt.

3. Verwendung gemäss Anspruch 3, dadurch gekennzeichnet, dass es sich um Gedächtnisschwäche handelt.

4. Verwendung gemäss Anspruch 1, dadurch gekennzeichnet, dass es sich um eine degenerative Demenz handelt.

5. Verwendung gemäss Anspruch 4, dadurch gekennzeichnet, dass es sich um Demenz vom Alzheimer-Typ oder um eine multi-infarktbedingte Demenz handelt.

## Claims

1. Use of p-chloro-N-(2-morpholinoethyl)benzamide for the manufacture of a medicament for the treatment or prevention of cognitive disorders.

2. Use in accordance with claim 1, characterized in that the cognitive disorder is caused by old age.

3. Use in accordance with claim 3, characterized in that the cognitive disorder is a hypomnesis.

4. Use in accordance with claim 1, characterized in that the cognitive disorder is a degenerative dementia.

5. Use in accordance with claim 4, characterized in that the degenerative dementia is a dementia of the Alzheimer type or a multi-infarct caused dementia.

## Revendications

1. Utilisation du p-chloro-N-(2-morpholinoéthyl)benzamide pour la préparation d'une composition pour le traitement ou la prévention de maladies cognitives.

2. Utilisation selon la revendication 1, caractérisée en ce qu'il s'agit d'une maladie cognitive due à l'âge.

3. Utilisation selon la revendication 1, caractérisée en ce qu'il s'agit de défaillance de la mémoire.

4. Utilisation selon la revendication 1, caractérisée en ce qu'il s'agit d'une démence dégénérative.

5. Utilisation selon la revendication 4, caractérisée en ce qu'il s'agit de démence du type Alzheimer ou d'une démence due à de multiples infarctus.
